# EUROPÄISCHE PATENTSCHRIFT

(11) **EP 2 094 212 B1**
(45) Veröffentlichungstag und Bekanntmachung des Hinweises auf die Patenterteilung: **10.08.2011**
(21) Anmeldenummer: 07816235.1
(22) Anmeldetag: 09.11.2007
(51) Int. Cl.: A61F 13/02, A61F 13/00

(54) **VORRICHTUNG ZUR WUNDBEHANDLUNG**
DEVICE FOR TREATING WOUNDS
DISPOSITIF DE TRAITEMENT DE PLAIE

(30) Priorität: 30.11.2006 CH 19442006
(43) Veröffentlichungstag der Anmeldung: 02.09.2009
(73) Patentinhaber: Medela Holding AG, 6340 Baar (CH)
(72) Erfinder: AICHER, Martin, 8049 Zürich (CH)
(74) Vertreter: Clerc, Natalia
(86) Internationale Anmeldenummer: PCT/CH2007/000552
(87) Internationale Veröffentlichungsnummer: WO 2008/064503

(56) Entgegenhaltungen:
- WO-A-00/78212
- US-A- 5 636 643
- US-A- 6 071 267

## Beschreibung

### Technisches Gebiet

Die Erfindung betrifft eine Vorrichtung zur Wundbehandlung gemäss Oberbegriff des Patentanspruchs 1.

### Stand der Technik

Offene Wunden, welche zu gross oder zu stark entzündet sind, um selbständig zu heilen, waren lange Zeit ein schwieriges Gebiet der Medizin. Es hat sich gezeigt, dass Wunddrainage unter Verwendung von Unterdruck die Heilung der Wunde stimuliert, unterstützt bzw. beschleunigt.

Dies ist beispielsweise in US 5 636 643 beschrieben. Dort ist eine Vorrichtung zur Wundbehandlung mit einer flüssigkeits- und gasdichten steifen Haube offenbart, welche über eine Wunde gelegt und ausserhalb der Wundränder auf die gesunde Haut befestigt wird. Unterhalb der Haube ist eine Wundauflage in Form eines Schaumstoffs auf bzw. in die Wunde gelegt. In der Haube wird von aussen mittels einer Vakuumpumpe ein Unterdruck erzeugt, um die Wundheilung zu beschleunigen.

Auch WO 03/018098 beschreibt eine Vorrichtung zur Wundbehandlung mit einer Haube und einem porösen Kissen, welches unter der Haube auf die Wunde gelegt ist. Hier wird vorgeschlagen, den Unterdruck automatisch zu oszillieren, um die Wundheilung zu stimulieren.

WO 2006/056408 schlägt vor, in die Haube Zufuhrvorrichtungen für Behandlungsmedien vorzusehen. Diese Behandlungsmedien werden zusammen mit dem Wundsekret durch eine Abfuhrvorrichtung abgeführt.

WO 2006/048246 offenbart einen Mehrkomponentenverband zur Wundbehandlung mittels Unterdruck. Dieser Verband weist superabsorbierende Polymere auf, wobei die absorbierten Wundsekrete an Polymere gebunden im Wundraum verbleiben.

Eine weitere Vorrichtung zur Wund behandlung wird in US 6011261 A beschrieben.

### Darstellung der Erfindung

Es ist deshalb eine Aufgabe der Erfindung, eine Vorrichtung zur Wundbehandlung zu schaffen, welche für unterschiedliche Wundgrössen einsetzbar ist.

Diese Aufgabe löst eine Vorrichtung zur Wundbehandlung mit den Merkmalen des Patentanspruchs 1.

Die erfindungsgemässe Vorrichtung zur Wundbehandlung eines Patienten mittels Unterdruck weist eine Wundauflage und eine Abdeckfolie auf, wobei die Abdeckfolie auf der Wundauflage angeordnet und mit dieser verbunden ist. Die Abdeckfolie ist vor Gebrauch mindestens teilweise gefaltet oder zusammengerollt.

Dadurch lässt sich die Wundauflage in eine für die zu behandelnde Wunde angepasste Form bringen, beispielsweise durch Zuschneiden, Brechen oder Abreissen, und anschliessend sofort auf die Wunde aufbringen. Da die Abdeck- und Befestigungsfolie bereits als integraler Bestandteil fest mit der Wundauflage verbunden ist, ist die Handhabung sehr einfach und für den Patient weniger schmerzhaft.

Vorzugsweise liegt ein Teil der Abdeckfolie vor Gebrauch flächig auf der Wundauflage auf. Vorzugsweise ist dies derjenige Teil, welcher den Drainageschlauch umgibt. Dies hat den Vorteil, dass die Abdeckfolie von einem klar definierten Gebiet aus entfaltet bzw. auseinander gerollt werden kann.

Vorzugsweise weist die Abdeckfolie vor Gebrauch eine kleinere Fläche auf als die Wundauflage. Dadurch muss lediglich die Wundauflage der Grösse der Wunde angepasst werden, nicht aber die Abdeckfolie. Sollte trotzdem eine Verkleinerung der Abdeckfolie notwendig sein, so kann dies nachträglich erfolgen, beispielsweise nachdem die Wundauflage in die Wunde eingebracht bzw. auf diese aufgelegt worden ist.

Vorzugsweise weist die Abdeckfolie ein Formgedächtnis auf und kann von einer Basisform in eine Gebrauchsform wechseln. Dadurch wird die Wunde kontrahiert.

Die erfindungsgemässe Vorrichtung kombiniert somit Wundbettfüllung, Wundversiegelung und Drainage auf einfachste Art und Weise.

Weitere vorteilhafte Ausführungsformen gehen aus den abhängigen Patentansprüchen hervor.

### Kurze Beschreibung der Zeichnungen

Im folgenden wird der Erfindungsgegenstand anhand eines bevorzugten Ausführungsbeispiels, welches in den beiliegenden Zeichnungen dargestellt ist, erläutert. Es zeigen:
Figur 1 einen Querschnitt durch eine erfindungsgemässe Vorrichtung in einer Basisform vor ihrer Verwendung und
Figur 2 einen Querschnitt durch die Vorrichtung gemäss Figur 1 bei ihrer Verwendung.

### Wege zur Ausführung der Erfindung

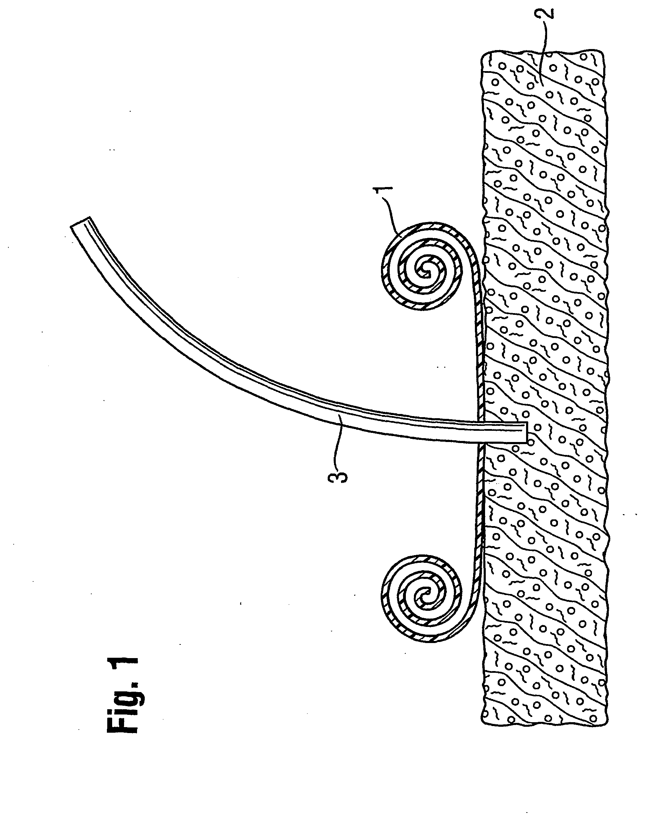

In Figur 1 ist eine erfindungsgemässe Vorrichtung zur Wundbehandlung mittels Unterdruck, d.h. zur Wunddrainage, dargestellt.

Die Vorrichtung weist eine Abdeckfolie 1, eine Wundauflage 2 und mindestens einen Schlauch, hier ein Drainageschlauch 3 auf. Der Drainageschlauch 3 durchdringt die Folie 1 und ragt in die Wundauflage 2 hinein.

Die Abdeckfolie 1 ist fest mit der Wundauflage 2 verbunden, bzw. sie ist ein integraler Bestandteil derselben. Sie kann beispielsweise aufgeklebt, mit dieser verschweisst oder formschlüssig verbunden sein.

Vor dem Gebrauch ist die Abdeckfolie 1 mindestens teilweise gefaltet oder zusammengerollt. In Figur 1 ist die Abdeckfolie 1 zusammengerollt. Die Breite der Abdeckfolie 1 ist vorzugsweise an die Breite der Wunde angepasst. Ein Teil der Folie 1 liegt jedoch auch vor Gebrauch flächig auf der Wundauflage 2 auf. Vorzugsweise ist es derjenige Teil der Abdeckfolie 1, welcher den Drainageschlauch 3 umgibt.

Die Abdeckfolie 1 kann rechteckig, insbesondere quadratisch, rund oder oval sein oder eine andere Form aufweisen. Vorzugsweise weist sie eine Form auf, welche der Grundfläche der Wundauflage 2 entspricht, wobei sie eine grössere Fläche aufweist als diese. Dabei sollte sie vorzugsweise so auf der Wundauflage 2 befestigt sein, dass sie diese auf dem gesamten Umriss derselben genügend überragt, um als Befestigungsfolie zu dienen.

Vor Gebrauch weist die Abdeckfolie 1 jedoch vorzugsweise eine kleinere Fläche auf als die Wundauflage 2, wie dies in Figur 1 sichtbar ist.

Vorzugsweise ist mindestens ein Teil der Abdeckfolie 1 selbsthaftend, damit sie im entrollten bzw. auseinander gefalteten Zustand auf die die Wunde umgebende gesunde Haut des Patienten befestigt werden kann. Der Teil ist vorzugsweise der gesamte Umrissbereich der Abdeckfolie 1. Es kann jedoch auch die gesamte, der Wundauflage 2 zugeneigten Oberfläche der Abdeckfolie 1 selbsthaftend ausgebildet sein, damit sie beim auseinander falten bzw. rollen gleich an der Wundauflage 2 haftet. Die Abdeckfolie 2 kann jedoch auch nicht selbsthaftend ausgebildet sein und mit einem zusätzlichen Klebemittel, beispielsweise einem Klebstreifen, auf der Haut befestigt werden.

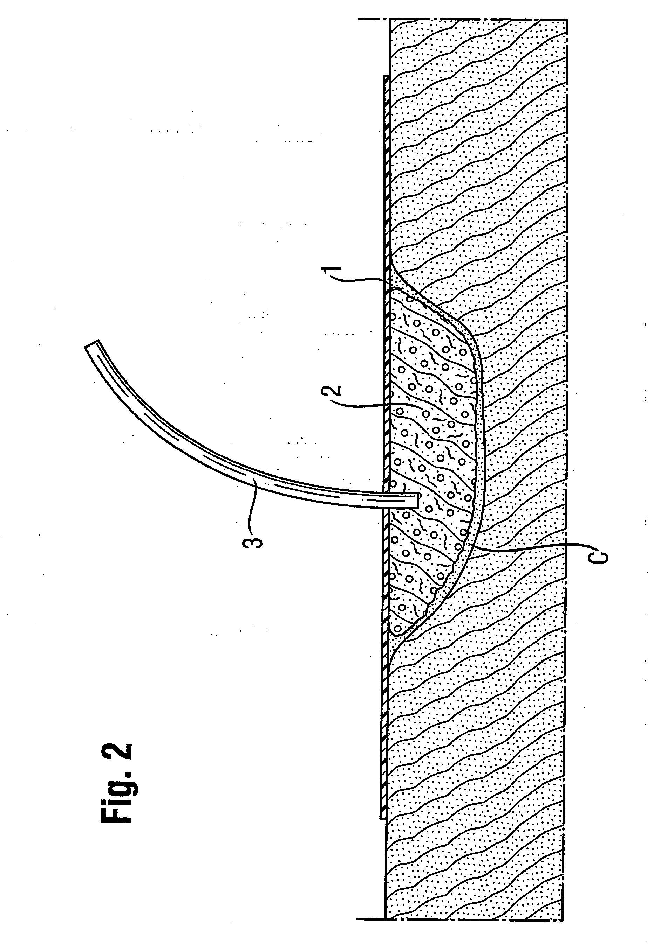

Die Abdeckfolie 1 und die Wundauflage 2 überdecken bzw. füllen somit die Kavität C der Wunde. Dies ist in Figur 2 dargestellt.

Die Wundauflage ist aus einem porösen, luft- und flüssigkeitsdurchlässigen Material gefertigt. Sie besteht vorzugsweise aus Textilien und kann insbesondere eine Dicke von 5 bis 80 mm aufweisen. Das Material kann auch ein Schwamm oder ein anderes geeignetes Material sein.

Die Abdeckfolie 1 ist aus einem luft- und flüssigkeitsdichten Material gefertigt. Sie kann aus einem Polymer bestehen. Vorzugsweise weist sie eine Dicke von 0.1 bis 5 mm auf.

Vorzugsweise weist die Abdeckfolie 1 ein Formgedächtnis auf. Sie kann hierfür beispielsweise aus einem entsprechenden Polymer, insbesondere aus Block-Copolymeren gefertigt sein. Andere geeignete Materialien mit Formgedächtnis sind Metalle, beispielsweise Nitinol.

Dank dem Formgedächtnis kann die Abdeckfolie 1 von einer Basisform in eine Gebrauchsform wechseln. Vorzugsweise findet dieser Wechsel bei einer normalen menschlichen Körpertemperatur statt. Bei Zimmertemperatur von circa 20° weist die Abdeckfolie 1 die Basisform auf. Der Wechsel kann jedoch auch bei Zimmertemperatur oder einer zwischen 20° und 37° liegenden Temperatur stattfinden. In diesem Fall ist die Abdeckfolie gekühlt zu lagern und im gekühlten Zustand aufzubringen, damit sie während der Aufbringung die Basisform aufweist. Formgedächtnispolymere in weiteren Varianten können auch durch Magnetismus oder Licht stimuliert werden.

Vorzugsweise kontraktiert bzw. kontrahiert die Gebrauchsform die Wunde und hält den Zug bzw. Druck während der Drainage aufrecht. Dies kann beispielsweise dadurch erfolgen, dass die Basisform plan ist und die Gebrauchsform kalottenförmig ist, wobei sich die Kalotte von der Wunde weg wölbt. Es ist jedoch auch möglich, dass sie sich lediglich zusammenzieht und so die Wunde kontrahiert.

Bei Verwendung der oben beschriebenen Vorrichtung wird die Wundauflage 2 entsprechend der Grösse der Wunde bzw. des Wundbetts verkleinert und an die Form des Wundbetts angepasst. Anschliessend wird sie in das Wundbett gelegt und die Abdeckfolie 1 auseinander gefaltet bzw. gerollt und auf der umliegenden Haut fixiert. Falls der Drainageschlauch 3 nicht schon in die Wundauflage 2 hineinragt, wird dieser nun eingesteckt und mit einer Vakuumpumpe verbunden. Es kann nun ein geeignetes Vakuum für die Wunddrainage angelegt werden. Das Vakuum kann intermittierend und oszillierend oder auf eine andere bekannte Weise angelegt werden. Es werden die im Stand der Technik bekannten Unterdrücke verwendet. Bei Entfernung der Vorrichtung lassen sich Abdeckfolie und Wundauflage je nach Ausführungsform gemeinsam oder einzeln entfernen.

Die erfindungsgemässe Vorrichtung ermöglicht eine einfache Anpassung an die Grösse und Form der Wunde und eine einfache Handhabung.

### Bezugszeichenliste

- 1: Abdeckfolie
- 2: Wundauflage
- 3: Drainageschlauch
- C: Kavität

## Patentansprüche

1. Vorrichtung zur Wundbehandlung eines Patienten mittels Unterdruck, wobei die Vorrichtung eine Wundauflage und eine Abdeckfolie aufweist, **dadurch gekennzeichnet, dass** die Abdeckfolie bereits vor Gebrauch der Vorrichtung auf der Wundauflage angeordnet und mit dieser verbunden ist, wobei die Abdeckfolie vor Gebrauch mindestens teilweise gefaltet oder zusammengerollt ist.

2. Vorrichtung nach Anspruch 1, wobei ein Teil der Abdeckfolie vor Gebrauch flächig auf der Wundauflage aufliegt.

3. Vorrichtung nach einem der Ansprüche 1 oder 2, wobei die Abdeckfolie vor Gebrauch im zusammengefalteten bzw. zusammengerollten Zustand eine kleinere Fläche aufweist als die Wundauflage.

4. Vorrichtung nach einem der Ansprüche 1 bis 3, wobei die Abdeckfolie auf die Wundauflage aufgeklebt, mit dieser verschweisst oder formschlüssig verbunden ist.

5. Vorrichtung nach einem der Ansprüche 1 bis 4, wobei die Vorrichtung mindestens einen Drainageschlauch umfasst, welcher die Abdeckfolie durchdringt und in die Wundauflage hineinragt, und wobei die Abdeckfolie im Bereich dieses Drainageschlauchs flächig auf der Wundauflage aufliegt.

6. Vorrichtung nach einem der Ansprüche 1 bis 5, wobei mindestens ein Teil des aufgerollten bzw. gefalteten Bereichs der Abdeckfolie selbsthaftend ist.

7. Vorrichtung nach einem der Ansprüche 1 bis 6, wobei die Abdeckfolie im auseinander gefalteten bzw. auseinander gerollten Zustand eine grössere Fläche aufweist als die Wundauflage, wobei sie dieser Wundauflage im gesamten Umfangsbereich vorsteht.

8. Vorrichtung nach einem der Ansprüche 1 bis 7, wobei die Wundauflage aus einem Textil besteht.

9. Vorrichtung nach einem der Ansprüche 1 bis 8, wobei die Abdeckfolie aus Polymer besteht.

10. Vorrichtung nach einem der Ansprüche 1 bis 8, wobei die Abdeckfolie ein Formgedächtnis aufweist und von einer Basisform in eine Gebrauchsform wechselt.

11. Vorrichtung nach Anspruch 10, wobei der Wechsel in die Gebrauchsform bei einer normalen menschlichen Körpertemperatur stattfindet.

12. Vorrichtung nach einem der Ansprüche 10 oder 11, wobei die Gebrauchsform die Wunde kontrahiert.

13. Vorrichtung nach einem der Ansprüche 10 bis 12, wobei die Gebrauchsform kalottenförmig ist, wobei sich dabei die Kalotte von der Wunde weg wölbt.

14. Vorrichtung nach einem der Ansprüche 10 bis 13, wobei die Abdeckfolie bei einer Temperatur, welche annähernd der Zimmertemperatur entspricht, die Basisform aufweist.

15. Vorrichtung nach einem der Ansprüche 10 bis 14, wobei die Abdeckfolie aus Block-Copolymer besteht.

16. Vorrichtung nach einem der Ansprüche 1 bis 15, wobei die Abdeckfolie eine Dicke von 0.1 bis 5 mm aufweist.

17. System zur Wunddrainage mit einer Vorrichtung zur Wundbehandlung gemäss einem der Ansprüche 1 bis 16 und einer damit verbindbaren Vakuumpumpe.

18. Verfahren zur Verwendung einer Vorrichtung gemäss einem der Ansprüche 1 bis 17, wobei die Länge und Breite der Wundauflage auf eine zu behandelnde Wunde angepasst wird, indem die Wundauflage verkleinert wird, die Wundauflage in bzw. auf die Wunde gelegt wird und die Abdeckfolie entfaltet bzw. auseinander gerollt und auf die der Wunde umgebende Haut fixiert wird.

## Claims

1. A device for treating wounds of a patient by means of low pressure, wherein the device comprises a wound dressing and a cover film, **characterized in that** the cover film is arranged on the wound dressing and connected thereto even before use of the device, wherein the cover film is at least partially folded or rolled up before use.

2. The device as claimed in claim 1, wherein a part of the cover film lies flat on the wound dressing before use.

3. The device as claimed in one of claims 1 and 2, wherein the cover film has a smaller surface area than the wound dressing when in the folded or rolled up state before use.

4. The device as claimed in one of claims 1 through 3, wherein the cover film is adhesively bonded on the wound dressing, welded to it or connected to it with a form fit.

5. The device as claimed in one of claims 1 through 4, wherein the device comprises at least one drainage tube that extends through the cover film and protrudes into the wound dressing, and wherein the cover film, in the area of this drainage tube, lies flat on the wound dressing.

6. The device as claimed in one of claims 1 through 5, wherein at least part of the rolled up or folded area of the cover film is self-adhesive.

7. The device as claimed in one of claims 1 through 6, wherein the cover film, in the state when unfolded or rolled out, has a greater surface area than the wound dressing and protrudes past this wound dressing about the entire circumference.

8. The device as claimed in one of claims 1 through 7, wherein the wound dressing is made from a textile.

9. The device as claimed in one of claims 1 through 8, wherein the cover film is made from polymer.

10. The device as claimed in one of claims 1 through 8, wherein the cover film has a shape memory and changes from a basic shape to a shape for use.

11. The device as claimed in claim 10, wherein the change to the shape for use takes place at a normal human body temperature.

12. The device as claimed in one of claims 10 and 11, wherein the shape for use contracts the wound.

13. The device as claimed in one of claims 10 through 12, wherein the shape for use has a dome-shaped configuration in which the dome curves away from the wound.

14. The device as claimed in one of claims 10 through 13, wherein the cover film has the basic shape at a temperature that corresponds approximately to room temperature.

15. The device as claimed in one of claims 10 through 14, wherein the cover film is made from a block copolymer.

16. The device as claimed in one of claims 1 through 15, wherein the cover film has a thickness of 0.1 to 5 mm.

17. A system for draining wounds, with a device for treating wounds as claimed in one of claims 1 through 16 and with a vacuum pump that can be connected to said device.

18. A method for using a device as claimed in one of claims 1 through 17, wherein the length and width of the wound dressing are adapted to a wound that is to be treated, by means of the wound dressing being made smaller, the wound dressing is placed into or onto the wound, and the cover film is unfolded or rolled out and is fixed on the skin surrounding the wound.

## Revendications

1. Dispositif de traitement de blessure d'un patient à l'aide d'une sous-pression, le dispositif comportant une surface d'appui de blessure et un film couvrant, **caractérisé en ce que** le film couvrant est déjà disposé sur la surface d'appui de blessure avant utilisation du dispositif et est relié à elle, le film couvrant étant au moins en partie plié ou enroulé avant utilisation.

2. Dispositif selon la revendication 1, une partie du film couvrant reposant à plat sur la surface d'appui de blessure avant utilisation.

3. Dispositif selon l'une quelconque des revendications 1 ou 2, le film couvrant comportant à l'état plié ou enroulé, avant utilisation, une surface inférieure à la surface d'appui de blessure.

4. Dispositif selon l'une quelconque des revendications 1 à 3, le film couvrant étant collé sur la surface d'appui de blessure, soudé à elle ou relié par complémentarité de formes.

5. Dispositif selon l'une quelconque des revendications 1 à 4, le dispositif comprenant au moins un tuyau de drainage qui traverse le film couvrant et ressort dans la surface d'appui de blessure et le film couvrant reposant à plat dans la zone de ce tuyau de drainage sur la surface d'appui de blessure.

6. Dispositif selon l'une quelconque des revendications 1 à 5, au moins une partie de la zone enroulée ou pliée du film couvrant étant autoadhésive.

7. Dispositif selon l'une quelconque des revendications 1 à 6, le film couvrant comportant à l'état déplié ou déroulé une surface plus importante que la surface d'appui de blessure du fait qu'elle surplombe cette surface d'appui de blessure sur l'ensemble de sa périphérie.

8. Dispositif selon l'une quelconque des revendications 1 à 7, la surface d'appui de blessure étant réalisée à partir d'un matériau textile.

9. Dispositif selon l'une quelconque des revendications 1 à 8, le film couvrant étant en polymère.

10. Dispositif selon l'une quelconque des revendications 1 à 8, le film couvrant étant doté d'une mémoire de forme et pouvant passer d'une forme de base à une forme d'utilisation.

11. Dispositif selon la revendication 10, le passage à la forme d'utilisation se produisant à la température normale d'un corps humain.

12. Dispositif selon l'une quelconque des revendications 10 ou 11, la forme d'utilisation contractant la blessure.

13. Dispositif selon l'une quelconque des revendications 10 à 12, la forme d'utilisation prenant une forme de calotte, la calotte étant bombée vers l'extérieur de la blessure.

14. Dispositif selon l'une quelconque des revendications 10 à 13, le film couvrant reprenant sa forme de base à une température correspondant approximativement à la température régnant dans une pièce normale.

15. Dispositif selon l'une quelconque des revendications 10 à 14, le film couvrant étant réalisé à partir d'un copolymère à blocs.

16. Dispositif selon l'une quelconque des revendications 1 à 15, le film couvrant présentant une épaisseur allant de 0,1 à 5 mm.

17. Système de drainage de blessure avec un dispositif de traitement de blessure selon l'une quelconque des revendications 1 à 16 et avec une pompe à vide pouvant y être reliée.

18. Procédé d'utilisation d'un dispositif selon l'une quelconque des revendications 1 à 17, la longueur et la largeur de la surface d'appui de blessure étant adaptées à la blessure à traiter en ce que la surface d'appui de blessure est réduite, que la surface d'appui de blessure est placée ou bien dans ou bien sur la blessure et que le film couvrant est déplié ou déroulé puis fixé sur la peau entourant la blessure.
